(19) Europäisches Patentamt
European Patent Office
Office européen des brevets

(11) **EP 4 600 215 A1**

(12) **EUROPEAN PATENT APPLICATION**
published in accordance with Art. 153(4) EPC

(43) Date of publication:
**13.08.2025 Bulletin 2025/33**

(21) Application number: 23874826.3

(22) Date of filing: **02.10.2023**

(51) International Patent Classification (IPC):
**C01G 25/00** *(2006.01)* **C09K 11/67** *(2006.01)*
**C09K 11/88** *(2006.01)*

(52) Cooperative Patent Classification (CPC):
**C01G 25/00; C09K 11/67; C09K 11/88**

(86) International application number:
**PCT/JP2023/035933**

(87) International publication number:
**WO 2024/075699 (11.04.2024 Gazette 2024/15)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.10.2022 JP 2022162690**

(71) Applicant: **Idemitsu Kosan Co.,Ltd.**
**Tokyo 100-8321 (JP)**

(72) Inventors:
• **KIMOTO, Yoshinori**
**Tokyo 100-8321 (JP)**
• **INATOME, Toru**
**Tokyo 100-8321 (JP)**
• **KATO, Takuya**
**Tokyo 100-8321 (JP)**

(74) Representative: **Hoffmann Eitle**
**Patent- und Rechtsanwälte PartmbB**
**Arabellastraße 30**
**81925 München (DE)**

(54) **CHALCOGENIDE PEROVSKITE**

(57)  A chalcogenide perovskite has an average particle size of less than 40 nm and an actual measured value of a band gap that matches a range of a band gap ($E_{g,bulk}$) unique to the semiconductor calculated by Formula (A) below based on a band gap calculated value ($E_{g,calc(HSE06)}$) obtained by a density functional theory using a hybrid functional (HSE06).
[Math. 1]

$$E_{g,bulk} = \left(0.9286 \times E_{g,calc(HSE06)} + 0.3049\right) \times (1 \pm 0.05) \quad \cdots (A)$$

**Description**

Technical Field

The present invention relates to a chalcogenide perovskite.

Background Art

In recent years, semiconductor light emitting materials have been widely used as light-emitting materials and wavelength conversion (color conversion, down-conversion) elements, and photoelectric conversion materials in photoelectric conversion elements. In these fields, materials are required to have high durability (for example, stability against water, oxygen, and heat) and high light absorption.

As means for solving the above problems, chalcogenide perovskites such as $BaZrS_3$ have been studied (see, for example, Non Patent Literature 1 and Non Patent Literature 2). The chalcogenide perovskites exhibit both excellent durability and a higher light absorption coefficient than conventional materials.

Citation List

Non Patent Literature

Non Patent Literature 1: V. K. Ravi et al., "Colloidal BaZrS3 chalcogenide perovskite nanocrystals for thin film device fabrication", The Royal Society of Chemistry, Nanoscale, 2021, Vol.13, p.1616-1623

Non Patent Literature 2: R. Yang et al., "Low-Temperature, Solution-Based Synthesis of Luminescent Chalcogenide Perovskite BaZrS3 Nanoparticles", J.Am. Chem. Soc. 2022, 144, 35, 15928-15931

Summary of Invention

In a case where a semiconductor material is used as a light emitting material, light emission can occur either by utilizing the band gap unique to the semiconductor material or by utilizing the quantum size effect.

As materials utilizing the quantum size effect, quantum dots (semiconductor nanocrystal particles) such as CdSe, InP, and halide perovskites are used. Quantum dots require precise size control to regulate the absorption and emission wavelengths, which results in high manufacturing costs. In addition, since the electronic energy levels of quantum dots become discrete compared to bulk materials, their light absorption coefficient decreases, making them less preferable not only as light emitting materials but also as photoelectric conversion elements.

On the other hand, in the case of a light emitting material using a band gap unique to a semiconductor material, since the particle size is larger than that of quantum dots, the stability of the dispersion liquid is lower, leading to issues such as aggregation and nozzle clogging, which can cause defects during manufacturing.

An object of the present invention is to provide a chalcogenide perovskite that emits light with a band gap unique to a semiconductor material while having a small particle size.

According to the present invention, the following chalcogenide perovskites and the like are provided.

1. A chalcogenide perovskite having an average particle size of less than 40 nm and an actual measured value of a band gap that matches a range of a band gap ($E_{g,bulk}$) unique to the semiconductor calculated by Formula (A) below based on a band gap calculated value ($E_{g,calc(HSE06)}$) obtained by a density functional theory using a hybrid functional (HSE06).
[Math. 1]

$$E_{g,bulk} = \left(0.9286 \times E_{g,calc(HSE06)} + 0.3049\right) \times \left(1 \pm 0.05\right) \quad \cdots (A)$$

2. The chalcogenide perovskite according to 1, in which the actual measured value of the band gap is more than 0 eV and less than 2.09 eV.

3. The chalcogenide perovskite according to 1 or 2, in which the actual measured value of the band gap is 1.0 eV or more and 2.0 eV or less.

4. The chalcogenide perovskite according to any one of 1 to 3, in which the actual measured value of the band gap is 1.8 eV or more and 2.0 eV or less.

5. The chalcogenide perovskite according to any one of 1 to 4, in which the average particle size is 4 nm or more and less than 40 nm.

6. The chalcogenide perovskite according to 5, in which the average particle size is 5 nm or more.

7. The chalcogenide perovskite according to 6, in which the average particle size is 6 nm or more.

8. The chalcogenide perovskite according to any one of 5 to 7, in which the average particle size is less than 35 nm.

9. The chalcogenide perovskite according to 8, in which the average particle size is less than 32 nm.

10. The chalcogenide perovskite according to 9, in which the average particle size is less than 30 nm.

11. The chalcogenide perovskite according to any one of 1 to 10, in which the chalcogenide perovskite has a composition represented by Formula (101) or (102) below:

$$ABCh_3 \quad ... \quad (101)$$

$$A'_2A_{n-1}B_nCh_{3n+1} \quad ... \quad (102)$$

(In Formulae (101) and (102), A and A each represent Ca, Mg, Sr, Ba, or a combination thereof; B represents Ti, Zr, Hf, or a combination thereof; and Ch represents S, Se, Te, or a combination thereof. In Formula (102), n is an integer of 1 or more and 10 or less. In Formula (102), A and A' may be the same as or different from each other. The chalcogenide perovskite may be a solid solution in which a part or all of A, A, B, and Ch are substituted with another element in the composition represented by Formula (101) or (102).)

12. The chalcogenide perovskite according to 11, in which n is 1 or more and 3 or less.

13. The chalcogenide perovskite according to claim 11 or 12, wherein a composition of the chalcogenide perovskite is $BaZrS_3$.

14. A powder including the chalcogenide perovskite according to any one of 1 to 13.

15. A solution including the chalcogenide perovskite according to any one of 1 to 13.

16. A thin film including the chalcogenide perovskite according to any one of 1 to 13.

17. A sheet including the chalcogenide perovskite according to any one of 1 to 13.

18. A device including the chalcogenide perovskite according to any one of 1 to 13.

19. The device according to 18, in which the device is a photoelectric conversion element.

According to the present invention, it is possible to provide a chalcogenide perovskite that emits light with a band gap unique to a semiconductor material while having a small particle size.

Brief Description of Drawings

Fig. 1 illustrates actual measured values and calculated values of a band gap of a chalcogenide perovskite.

Fig. 2 illustrates a regression line created based on the actual measured value and the calculated value of the band gap of the chalcogenide perovskite.

Fig. 3 illustrates an X-ray diffraction spectrum of a synthetic substance obtained in Example 1.

Fig. 4 is a STEM image (magnification: 200,000) of the synthetic substance obtained in Example 1.

Fig. 5 is a STEM image (magnification: 400,000) of the synthetic substance obtained in Example 1.

Fig. 6 is a fluorescence spectrum of the synthetic substance obtained in Example 1.

Description of Embodiments

Hereinafter, embodiments for carrying out the invention will be described.

In the present specification, "x to y" represents a numerical range of "x or more and y or less". The upper limit and the lower limit described for the numerical range can be arbitrarily combined.

Among the individual embodiments of the aspects according to the present invention described below, it is possible to combine two or more embodiments that do not contradict each other, and an embodiment in which two or more embodiments are combined is also an embodiment of the aspect according to the present invention.

The chalcogenide perovskite according to one aspect of the present invention emits light with a band gap unique to a semiconductor, and has an average particle size of less than 40 nm.

Here, the band gap unique to the semiconductor will be described. The band gap unique to the semiconductor can be relatively accurately obtained by a density functional theory using Heyd-Scuseria-Emzerhof (HSE06) which is a hybrid functional. However, it has been reported that the band gap of the chalcogenide perovskite calculated by DFT is smaller than the actual measured value (see Literature 1 to Literature 3 below).

1. Y Nishigaki et al., "Extraordinary Strong Band-Edge Absorption in Distorted Chalcogenide Perovskites", Sol. RPL. 2020, 1900555

2. S. Sharma et al., "Bandgap Tuning in BaZrS3 Perovskite Thin Films", ACS Appl. Electron. Mater. 2021, 3, 8, 3306-3312

3. K Hanazawa et al., "Material Design of Green-Light-Emitting Semiconductors: Perovskite-Type Sulfide SrHfS3", J. Am. Chem. Soc. 2022, 141, 13, 5343-5349

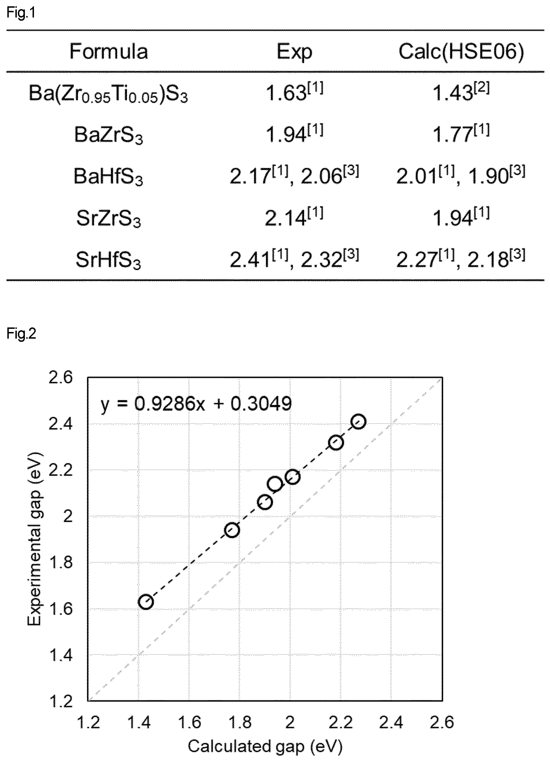

Fig. 1 illustrates a calculated value and an actual measured value of band gaps of each chalcogenide perovskite reported so far. Fig. 2 illustrates a regression line created by plotting the measured value on the vertical axis and the calculated value on the horizontal axis using the calculated value and the measured value of the band gap in each chalcogenide perovskite in Fig. 1.

Note that [1] in Fig. 1 indicates the value of Literature 1 above, [2] indicates the value of Literature 2 above, and [3] indicates the value of Literature 3 above. The unit is eV.

In Fig. 1, there are two actual measured values and two calculated values for $BaHfS_3$ and $SrHfS_3$, respectively and it can be seen that there is a discrepancy of approximately 5% between each pair of values. The factors contributing to this discrepancy include differences in samples among literature sources, variations in fitting methods used to calculate the band gap from diffuse reflection spectra, and differences in DFT calculation conditions. From this, there is a possibility that an error occurs between the value obtained by the regression line using the calculated value ($E_{g,calc(HSE06)}$) of the band gap obtained by the density functional theory using the hybrid functional (HSE06) and the actual measured value. Therefore, a range obtained by adding the measurement error $\pm 5\%$ to the value calculated using the regression line, that is, $E_{g,bulk}$ calculated by Formula (A) below is set as the band gap unique to the semiconductor.

[Math. 2]

$$E_{g,bulk} = \left(0.9286 \times E_{g,calc(HSE06)} + 0.3049\right) \times \left(1 \pm 0.05\right) \quad \cdots (A)$$

(In the formula, $E_{g,calc(HSE06)}$ is a calculated value of a band gap obtained by a density functional theory using a hybrid functional (HSE06).)

The fact that the actual measured value of the band gap is included in the range of the value obtained from Formula (A) above means that the light emission of the chalcogenide perovskite is not due to the quantum size effect but is the light emission of the band gap unique to the semiconductor material.

In the present application, the actual measured value of the band gap can be measured based on a fluorescence spectrum or a diffuse reflection spectrum. In the case of measurement based on a fluorescence spectrum, the fluorescence spectrum is measured, and the measured light emission peak energy corresponds to the actual measured value of the band gap. Note that measurement of the band gap based on the diffuse reflection spectrum may cause an error due to fitting, and thus measurement based on the fluorescence spectrum is more preferable.

In one embodiment, the actual measured value of the band gap is more than 0 eV and less than 2.09 eV. The actual measured value of the band gap is 1.0 eV or more, 1.5 eV or more, 1.7 eV or more, or 1.8 eV or more. The actual measured value of the band gap is 2.05 eV or less, 2.0 eV or less, or 1.98 eV or less.

The chalcogenide perovskite of the present aspect has an average particle size of less than 40 nm. As a result, the dispersion liquid has good stability even though the particles emit light with a band gap unique to the semiconductor material, and it is possible to suppress defects such as aggregation and nozzle clogging at the time of producing.

In one embodiment, the average particle size is 4 nm or more, 5 nm or more, or 6 nm or more. As a result, the light emission is not caused by the quantum size effect, but is likely to be caused by the light emission with the band gap unique to the semiconductor material, so that the particle size dependence of the emission wavelength can be eliminated.

In addition, the average particle size is less than 35 nm, less than 32 nm, or less than 30 nm. The particle size can be appropriately adjusted depending on the use form of the chalcogenide perovskite particles.

In the present application, the average particle size is measured using a microscope method on the dispersion liquid. Details will be described in examples.

By covering the chalcogenide perovskite of the present aspect with a material having a band gap larger than that of the chalcogenide perovskite (core-shell structure), the carrier confinement effect can be enhanced.

In the case of having a core-shell structure, the absorption edge may be shifted due to absorption of the shell material, and thus it is preferable to measure the band gap based on the fluorescence spectrum instead of the diffuse reflection spectrum.

The chalcogenide perovskite according to one embodiment is a compound containing chalcogen (Group 16 elements (S, Se, or Te) other than oxygen) having a perovskite type crystal structure, and contains, in addition to chalcogen, two or more kinds of metal elements such as transition metal elements and alkaline earth metal elements as constituent components of the perovskite type crystal structure.

In one embodiment, the chalcogenide perovskite has, for example, a composition represented by Formula (101) or (102) below.

$$ABCh_3 \dots \qquad (101)$$

$$A'_2A_{n-1}B_nCh_{3n+1} \dots \qquad (102)$$

In Formulae (101) and (102) above, A and A each represent Mg, Ca, Sr, Ba, or a combination thereof in any ratio, B represents Ti, Zr, Hf, or a combination thereof in any ratio, and Ch represents S, Se, Te, or a combination thereof in any ratio.

In Formula (102), n is an integer of 1 or more and 10 or less. n is preferably 1 or more and 3 or less.

In Formula (102), A and A may be the same as or different from each other.

In one embodiment, the chalcogenide perovskite may be a solid solution in which a part or all of A, A, B, and Ch are substituted with another element in the composition represented by Formula (101) or (102).

The chalcogenide perovskite (ABCh$_3$) represented by Formula (101) has a crystal structure of, for example, a cubic perovskite, a tetragonal perovskite, an orthorhombic perovskite, or a double perovskite.

The chalcogenide perovskite (A'$_2$A$_{n-1}$B$_n$Ch$_{3n+1}$) represented by Formula (102) has, for example, a crystal structure of a Ruddlesden-Popper layered perovskite.

In one embodiment, the composition of the chalcogenide perovskite is preferably BaZrS$_3$.

In one embodiment, the chalcogenide perovskite is surface-modified with a ligand.

The chalcogenide perovskite of the present embodiment surface-modified with a ligand can be easily dispersed in a dispersion medium such as a solvent or a polymer compound.

There is no limitation on the type of the ligand surface-modifying the chalcogenide perovskite, and it is sufficient that the ligand is generally used for dispersing nanoparticles. Examples of the surface-modifying ligand include oleylamine, oleic acid, dodecanethiol, and trioctylphosphine.

The dispersion medium may be a liquid or a solid.

The liquid dispersion medium may be, for example, various solvents selected from the group consisting of nonaqueous solvents such as toluene and hexane and water, or a solution in which components other than the chalcogenide perovskite are dispersed in these solvents.

The solid dispersion medium may be, for example, a polymer compound such as polyethylene.

For example, in order to disperse the chalcogenide perovskite synthesized by a solid phase synthesis method in the above-described dispersion medium, it is necessary to improve dispersibility in various dispersion media by performing surface modification of modifying the surface of the chalcogenide perovskite with a ligand.

On the other hand, in a case where a chalcogenide perovskite is synthesized by a liquid phase synthesis method, a synthesis reaction is performed in a state in which a ligand is present in advance in a liquid phase to be a reaction liquid for liquid phase synthesis, whereby a chalcogenide perovskite can be obtained as particles surface-modified with the ligand.

In the composition according to an embodiment, the chalcogenide perovskite according to the present aspect, which is surface-modified with a ligand, is dispersed in a dispersion medium.

As described above, the particles of the chalcogenide perovskite surface-modified with the ligand can be easily dispersed in a dispersion medium such as a solvent or a polymer compound. Therefore, in the composition according to the present embodiment, the chalcogenide perovskite is uniformly dispersed in the dispersion medium, and the composition has good characteristics as a composition.

The chalcogenide perovskite according to the present aspect can be synthesized, for example, by the synthesis method according to an embodiment of the present invention, that is, by reacting a complex having a first metal atom and a complex having a second metal atom, which have a ligand that does not contain an oxygen atom (O) and a halogen atom as coordination atoms, in a liquid phase, setting the temperature of the liquid phase to 120°C to 450°C, and setting the reaction time to 0 seconds or longer. Note that "0 seconds" refers to the reaction start time point of the complex having the first metal atom and the reaction start time point of the complex having the second metal atom, for example, in the hot injection method, a time point at which the reaction start time point of the complex having the first metal atom and the reaction start time point of the complex having the second metal atom are aligned in the liquid phase, and in the heat-up method, a time point at which the liquid phase reaches a predetermined temperature.

The present inventors have found that in a case where a chalcogenide perovskite is synthesized in a liquid phase, for example, if a component containing a halogen atom such as a halide or a component containing an oxygen atom (O) such as an acetate is mixed during liquid phase synthesis, the reaction may be inhibited, and the purity and crystal structure of the chalcogenide perovskite as an object are affected.

Examples of the halogen atom include a fluorine atom (F), a chlorine atom (CI), a bromine atom (Br), and an iodine atom (I).

In the present aspect, by reacting the complex having the first metal atom and the complex having the second metal atom, which have a ligand not containing an oxygen atom and a halogen atom as a coordination atom, in the liquid phase, the liquid phase synthesis reaction can be allowed to proceed without causing, for example, a component containing an oxygen atom such as a metal acetate or a component containing a halogen atom such as a metal halide to be present in the liquid phase. This makes it possible to suppress the reaction between the constituent element of the chalcogenide perovskite, and the oxygen atom and the halogen atom in the liquid phase. As a result, a crystal structure similar to that of the solid phase synthesis can be expressed.

In the present aspect, the band gap of the obtained chalcogenide perovskite can be adjusted, for example, by controlling the composition of the chalcogenide perovskite according to the constituent elements and blending amounts of the raw materials to be used. For example, when the composition of the chalcogenide perovskite is $BaZrS_3$, the measured value of the band gap is 1.96 eV (see Example 1).

The average particle size of the obtained chalcogenide perovskite can be controlled, for example, by adjusting the concentration of each complex in the liquid phase or the reaction time.

Hereinafter, a complex having a first metal atom and a complex having a second metal atom, which are starting materials, and a synthesis method of a chalcogenide perovskite will be described.

[Complex having First Metal Atom and Complex having Second Metal Atom]

The ligands of the complex having a first metal atom and the complex having a second metal atom used in the present aspect are not particularly limited, except that the ligands do not contain an oxygen atom and a halogen atom as coordination atoms. Examples thereof include a ligand coordinated with an atom selected from the group consisting of a nitrogen atom (N), a sulfur atom (S), a selenium atom (Se), a tellurium atom (Te), a carbon atom (C), and a phosphorus atom (P).

Examples of a ligand coordinated with a nitrogen atom include alkylamines ($NR_2$) such as amine, dimethylamine, diethylamine, and methylethylamine; arylamines (NHAr) such as phenylamine; trialkylsilylamines ($N(SiR_3)_2$) such as silylamine and trimethylsilylamine; and nitrogen-containing aromatic rings such as pyrazole.

Examples of the ligand coordinated with a sulfur atom include dithiocarbamate ($S_2CNR_2$), xanthate ($S_2COR$), trithiocarbamate ($S_2CSR$), dithioester ($S_2CR$), and thiolate (SR).

Examples of the ligand coordinated with a selenium atom or a tellurium atom include compounds in which some or all of the sulfur atoms of the ligand coordinated with the sulfur atoms are substituted with a selenium atom or a tellurium atom.

The complex having the first metal atom and the complex having the second metal atom may be a single compound (polynuclear complex). Preferably, the first metal atom and the second metal atom may be bonded via a ligand coordinated with a sulfur atom, a selenium atom, or a tellurium atom.

Examples of the polynuclear complex in which the ligand is coordinated with a sulfur atom include heterobimetallic thiolates and heterobimetallic sulfides.

Examples of the polynuclear complex in which the ligand is coordinated with a selenium atom or a tellurium atom include a polynuclear complex in which a part or all of sulfur atoms in the polynuclear complex in which the ligand is coordinated with sulfur atoms are substituted with selenium atoms or tellurium atoms.

Examples of ligands that coordinate via a carbon atom include alkanes such as methyl and ethyl; unsaturated hydrocarbon rings such as cyclopentadiene, cyclooctadiene, and indene; groups derived from benzyl or the aforementioned unsaturated hydrocarbon rings; and cyano (CN).

Examples of the ligand coordinated with a phosphorus atom include trialkylphosphines ($PR_3$) such as trioctylphosphine and tricyclohexylphosphine; triarylphosphine ($PAr_3$) such as triphenylphosphine and tri(o-tolyl) phosphine; and diphosphine ($R_2P$-$(CH_2)_m$-$PR_2$).

The ligand may be a derivative of the ligand described above.

In each of the general formulas described above, R represents a hydrogen atom, a saturated hydrocarbon group having 1 to 20 carbon atoms, or an unsaturated hydrocarbon. In a case where one ligand contains two or more Rs, R may be the same as or different from each other.

Ar is an aryl group having 6 to 20 carbon atoms which may have a substituent, such as a phenyl group, a naphthyl group, or an anthracenyl group. Examples of the substituent include an alkyl group having 1 to 10 carbon atoms. In a case where one ligand contains two or more Ars, Ar may be the same as or different from each other.

m is an integer of 1 to 10.

Among the ligands described above, a ligand coordinated with a nitrogen atom or a ligand coordinated with a sulfur atom is preferable.

In addition, a ligand having a structure selected from the group consisting of Formulae (1) to (7) below is

preferable.

$$S_2CNR^a_2 \qquad (1)$$

$$S_2COR \qquad (2)$$

$$S_2CSR^a \qquad (3)$$

$$S_2CR^a \qquad (4)$$

$$SR^a \qquad (5)$$

$$NR^a_2 \qquad (6)$$

$$N(SiR^a_3)_2 \qquad (7)$$

(In the formula, $R^a$ represents a hydrogen atom, a saturated hydrocarbon group having 1 to 20 carbon atoms, or an unsaturated hydrocarbon group having 1 to 20 carbon atoms.) In a case where a single ligand has a plurality of $R^a$ groups, the plurality of $R^a$ groups may be the same or different.)

Examples of the saturated hydrocarbon group and the unsaturated hydrocarbon include an alkyl group having 1 to 20 carbon atoms, a cycloalkyl group having 3 to 20 carbon atoms, an alkenyl group having 2 to 20 carbon atoms, an alkynyl group having 2 to 20 carbon atoms, a cycloalkenyl group having 3 to 20 carbon atoms, a cycloalkynyl group having 3 to 20 carbon atoms, a cycloalkadienyl group having 3 to 20 carbon atoms, and an aryl group having 6 to 20 carbon atoms.

The saturated hydrocarbon group and the unsaturated hydrocarbon may have a substituent. Examples of the substituent include an alkyl group having 1 to 10 carbon atoms.

In one embodiment, the ligand is preferably a compound represented by Formula (1) above. In addition, $R^a$ in Formula (1) is preferably an alkyl group having 1 to 10 carbon atoms, and more preferably an alkyl group having 2 to 6 carbon atoms.

The ligand used in the present aspect can be synthesized according to a conventional method. In addition, a commercially available compound may be used.

The metal atom of the complex having the first metal atom is a metal atom selected from alkaline earth metal elements, and the metal atom of the complex having the second metal atom is a metal atom selected from transition metal elements.

In one embodiment, the metal atom of the complex having the first metal atom is at least one of Mg, Ca, Sr, and Ba, and the metal atom of the complex having the second metal atom is at least one of Ti, Zr, and Hf.

The complex having a first metal atom and the complex having a second metal atom can be synthesized by a known method by selecting raw material components according to the types of metal atoms and ligands each having. In addition, a commercially available metal complex may be used.

Hereinafter, as an example of a synthesis method of a complex having a first metal atom and a complex having a second metal atom, synthesis of a metal complex ($M(S_2CNR_2)_X$) having a ligand ($S_2CNR_2$) represented by Formula (1) will be described.

First, at least one selected from, for example, Ba, Sr, Ca, Mg, Ti, Zr, and Hf or a salt thereof is dissolved in a solvent such as water as a metal raw material (M) that is a supply source of metal atoms of the metal complex.

The solvent is not particularly limited as long as it can dissolve the metal raw material (M), and may be, for example, an organic solvent.

Next, ammonia or an amine compound is added to the solution in which the metal raw material is dissolved, serving as a nitrogen (N) supply source for the complex ligand to be synthesized, followed by stirring.

As the amine compound, a primary amine or a secondary amine can be used without particular limitation, and the type of the substituted hydrocarbon group and the like in these amine compounds is also not particularly limited.

Next, a supply source of sulfur (S) contained in the complex ligand to be synthesized is further added to the solution to which the nitrogen (N) supply source has been added and stirred.

Typical examples of the supply source of the sulfur (S) include $CS_2$, but other compounds can be used depending on the composition of the complex ligand to be synthesized. In a case where Se or Te is contained instead of the sulfur (S), $CSe_2$ or $CTe_2$ can be used.

Next, the desired metal complex can be obtained by isolating the solid produced by stirring or the precipitate precipitated in the solution by a known method.

[Synthesis Method of Chalcogenide Perovskite]

In the present aspect, a chalcogenide perovskite is synthesized by reacting the complex having a first metal atom and the complex having a second metal atom described above in a solvent.

The solvent can be appropriately selected from the viewpoints of solubility of the complex having the first metal atom and the complex having the second metal atom, $H_2S$ generation efficiency, and the like.

Examples of the solvent include saturated aliphatic hydrocarbons, unsaturated aliphatic hydrocarbons, aromatic hydrocarbons, nitrogen-containing heterocyclic compounds, alcohols, aldehydes, carboxylic acids, primary amines, secondary amines, tertiary amines, phosphine compounds, and thiols.

As the solvent, a solvent having a higher boiling point is preferably used in consideration of the fact that the liquid phase may be heated to a high temperature of, for example, 300°C or higher in the liquid phase synthesis stage described later.

For example, an alkene having 10 to 30 carbon atoms such as octadecene has a high boiling point, and thus can be suitably used as a solvent.

In one embodiment, in addition to the complex having the first metal atom and the complex having the second metal atom, a chalcogen compound can be further added to the liquid phase. For example, in a case where the ligands of the complex having a first metal atom and the complex having a second metal atom do not have a chalcogen element such as the sulfur (S) or are insufficient with the chalcogen element of the ligand, the composition of the chalcogenide perovskite as an object can be adjusted by adding the chalcogen compound to the liquid phase.

As the chalcogen compound, a chalcogen compound that does not contain an oxygen atom (O) and a halogen atom is preferably used. For example, sulfur or a sulfide such as carbon disulfide ($CS_2$) or hydrogen sulfide ($H_2S$), selenium or a selenide, tellurium or a telluride, hexanethiol, octanethiol, decanethiol, dodecanethiol (n-dodecanethiol, t-dodecanethiol), hexadecanethiol, mercaptopropylsilane, a thiourea compound ($S=C(NR_2)_2$), sulfur-trioctylphosphine (trioctyl-phosphine sulfide; S-TOP), sulfur-tributylphosphine (S-TBP), sulfur-triphenylphosphine (S-TPP), sulfur-trioctylamine (S-TOA), bis(trimethylsilyl) sulfide, ammonium sulfide, sodium sulfide, selenium-trioctylphosphine (Se-TOP), selenium-tributylphosphine (Se-TBP), and selenium-triphenylphosphine.

Among them, the thiourea compound ($S=C(NR_2)$ has a boiling point higher than that of, for example, carbon disulfide ($CS_2$), and thus can be suitably used as a chalcogen compound.

In one embodiment, in addition to the complex having the first metal atom and the complex having the second metal atom described above, an amine-based compound can be further added to the liquid phase. The amine-based compound functions as a reaction initiator. The amine-based compound may be used as the solvent described above.

As the amine-based compound, an amine-based compound that does not contain an oxygen atom (O) and a halogen atom is preferably used. Examples thereof include alkylamines having one alkyl group such as oleylamine, hexadecylamine, and octadecylamine, dialkylamines having two alkyl groups having 1 to 30 carbon atoms, and trialkylamines having three alkyl groups having 1 to 30 carbon atoms (for example, trioctylamine).

From the viewpoint of sufficiently exhibiting the function as a reaction initiator between the ligand and the metal component, it is preferable to add the amine-based compound in an amount equal to or more than the total amount of the number of ligands contained in the complex having the first metal atom and the complex having the second metal atom added to the liquid phase.

In addition, from the viewpoint of exhibiting a function as a surfactant attached to the surface of the finally obtained chalcogenide perovskite particle, an amine-based compound may be added to the liquid phase in excess of the aforementioned amount (equivalent to the total number of ligands).

In the synthesis method of the present aspect, the complex having the first metal atom and the complex having the second metal atom described above, and the chalcogen compound and the amine-based compound as necessary may be reacted in a liquid phase, and the procedure and the like are not particularly limited. The synthesis of the present aspect can be performed by a known method such as a hot injection method, a heat-up method, or a flow synthesis method.

The blending ratio of the complex having the first metal atom and the complex having the second metal atom can be adjusted according to the composition of the chalcogenide perovskite as an object.

In the synthesis method according to one embodiment, when the raw material components include at least a complex containing a first metal atom, a complex having a second metal atom, and a solvent, a first raw material having at least the solvent among the raw material components is prepared, along with a second raw material containing at least the remaining raw material components that are not included in the first raw material. Then, the reaction is caused by adding the second raw material to the first raw material.

In the present embodiment, it is preferable to add the second raw material to the first raw material in a state in which at least one of the first raw material and the second raw material is heated in advance.

In addition, the second raw material may be added to the first raw material and then heated to cause a reaction.

Hereinafter, as the synthesis method of the present aspect, a case where the synthesis is performed using a hot injection method will be described as an example.

First, the first raw material is heated in advance. The heating rate is not particularly limited, and heating is performed at a heating rate of, for example, 1 to 30°C/min, typically 1 to 10°C/min.

The temperature of the first raw material after heating is not particularly limited, and may be, for example, room temperature to 450°C or 120°C to 360°C.

Next, a second raw material is added to the first raw material to cause a reaction between the complex having the first metal atom and the complex having the second metal atom. Similarly to the first raw material, the second raw material may be heated in advance. The addition rate of the second raw material is not particularly limited, but is preferably adjusted in consideration of the temperature decrease of the liquid phase due to the addition of the second raw material.

The liquid phase temperature after mixing is 120°C to 450°C, preferably 120°C to 400°C, and more preferably 220°C to 360°C.

For example, in a case where either the complex having a first metal atom or the complex having a second metal atom has the ligand (dithiocarbamate ($S_2CNR_2$)) represented by Formula (1) described above, if the temperature of the liquid phase is 120°C or higher, the metal complex having the ligand can start the reaction with the amine-based compound, and thus the reaction between the complex having the first metal atom and the complex having the second metal atom proceeds smoothly. The reaction can be started without using an amine-based compound, but in this case, the temperature of the liquid phase is preferably higher than 120°C.

In addition, when the temperature of the liquid phase is 360°C or lower, vaporization of a commonly used solvent is suppressed, and stable liquid phase synthesis can be performed.

In the present embodiment, by adding the second raw material to the first raw material in a state in which the first raw material is heated in advance, when all the components (solvent, complex having a first metal atom, and complex having a second metal atom) contributing to the synthesis of the chalcogenide perovskite are aligned in the liquid phase, all of them are in a high temperature state at once, so that the reaction start time point of the complex having the first metal atom and the reaction start time point of the complex having the second metal atom can be substantially simultaneously performed. As a result, the reaction of the complex having the first metal atom and the reaction of the complex having the second metal atom proceed in a well-balanced manner, and the chalcogenide perovskite can be synthesized with high purity.

In a case where the above-described amine-based compound is added to the liquid phase as the reaction initiator, the raw material component includes a solvent, a complex having a first metal atom, a complex having a second metal atom, and an amine-based compound. The combination of components used as the first raw material and the second raw material is not particularly limited.

For example, the first raw material may be a solvent alone, and the second raw material may be a mixture of a complex having a first metal atom, a complex having a second metal atom, and an amine-based compound.

In addition, the first raw material may be a mixture of a solvent and an amine-based compound, and the second raw material may be a mixture of a complex having a first metal atom and a complex having a second metal atom.

In addition, the first raw material may be a mixture of one of a complex having a first metal atom or a complex having a second metal atom and a solvent, and the second raw material may be a mixture of the other of the complex having the first metal atom or the complex having the second metal atom and an amine-based compound.

In a case where the chalcogen compound described above is added to the liquid phase, the raw material component includes a solvent, a complex having a first metal atom, a complex having a second metal atom, and a chalcogen compound.

In this case, the combination of components used as the first raw material and the second raw material is not particularly limited.

For example, the first raw material may be a solvent alone, and the second raw material may be a mixture of a complex having a first metal atom, a complex having a second metal atom, and a chalcogen compound.

In addition, the first raw material may be a mixture of a solvent and a chalcogen compound, and the second raw material may be a mixture of a complex having a first metal atom and a complex having a second metal atom.

In addition, the first raw material may be a mixture of one of a complex having a first metal atom or a complex having a second metal atom and a solvent, and the second raw material may be a mixture of the other of the complex having the first metal atom or the complex having the second metal atom and a chalcogen compound.

The liquid phase obtained by adding the second raw material to the first raw material is heated and stirred for 0 seconds or longer, and preferably 10 hours or longer, and 24 hours or shorter in a state of being maintained at a temperature of 120°C to 450°C, preferably 120°C to 400°C, and more preferably 220°C to 360°C. After completion of stirring, the precipitate precipitated in the liquid phase is isolated by a known method to obtain chalcogenide perovskite particles as an object.

In the series of liquid phase synthesis described above, it is preferable to perform the synthesis in a state in which components such as water and oxygen are removed as much as possible so that these components do not exist in the synthesis atmosphere. For example, the series of liquid phase synthesis described above is preferably performed under an inert atmosphere filled with nitrogen or argon.

The synthesis of the present aspect may be performed, for example, by a flow synthesis method described below.

First, a complex having a first metal atom, a complex having a second metal atom, and at least one of a chalcogen compound and an amine-based compound are each dissolved in a solvent, and separately circulated as a raw material flow of three lines or four lines.

Next, the reaction is started by merging all the raw material flows simultaneously or stepwise. At this time, it is preferable to heat at least one of the raw material flows in advance before merging. The raw material flow heated in advance is preferably a flow having the largest flow rate. In addition, since the flow synthesis has a small reaction field, the temperature can be instantaneously raised even when heating is performed immediately after the merging of the raw material flows, and the reaction start time point of the complex having the first metal atom and the reaction start time point of the complex having the second metal atom can be substantially simultaneously performed. The heating rate at the time of heating and the temperature of the component after heating are the same as those in the hot injection method. The following processes are the same as those in the hot injection method.

According to the flow synthesis method, since the temperature of the component contributing to the synthesis of the chalcogenide perovskite can be raised in a short time after merging, the reaction start time point of the complex having the first metal atom and the reaction start time point of the complex having the second metal atom can be substantially simultaneously performed. In addition, since the flow synthesis has a small reaction field, the collision probability between the raw materials, that is, the reaction efficiency is high. As a result, the reaction of the complex having the first metal atom and the reaction of the complex having the second metal atom proceed in a well-balanced manner, and the chalcogenide perovskite can be synthesized with high purity.

In addition, the yield of the chalcogenide perovskite increases, and the energy cost can be reduced. In addition, since the waste amount of impurities contained in the final synthetic substances can be reduced, the environmental load can be reduced. In addition, since it is possible to prevent an accident such as an explosion on a manufacturing line caused by impurities, safety in manufacturing is enhanced.

In the synthesis method of the present aspect described above, chalcogenide perovskite particles can be obtained without performing the firing process. In general, when the firing process is performed, nano-sized primary particles are aggregated to form secondary particles, but the formation of such secondary particles is suppressed by not performing the firing process. Therefore, fine chalcogenide perovskite nanoparticles can be obtained.

In addition, since the synthesis method of the present aspect is a liquid phase synthesis method, impurities are less likely to be mixed in the particles in the synthesis process as compared with the solid phase synthesis method. Since the liquid phase synthesis method is characterized by uniform nucleation and particle growth from a liquid phase, it is possible to obtain fine nanoparticles having a narrow particle size distribution by preventing aggregation of nuclei generated in the liquid phase (see, for example, "Kiyoshi Kanie et al., 'Liquid Phase Synthesis of Functional Inorganic Nanoparticles Controlled in Size and Shape and Application to Hybrid Materials by the Surface Modification", Journal of the Japanese Association for Crystal Growth, 2017, Vol. 44, No. 2, p. 66-73").

Therefore, according to the synthesis method of the present aspect, it is possible to obtain chalcogenide perovskite nanoparticles that are less contaminated with impurities in the particles and have a narrow and uniform particle size distribution.

Examples of the product form of the chalcogenide perovskite nanoparticles include a powder, a solution, an ink, a resin, a thin film, and a sheet. In addition, as an application example, application to various devices such as a light emitting device and a photoelectric conversion element is assumed.

(Powder)

The powder is in a state in which chalcogenide perovskite nanoparticles are aggregated. Hereinafter, the chalcogenide perovskite nanoparticles are sometimes referred to as primary particles, and the chalcogenide perovskite nanoparticles in an aggregated state are sometimes referred to as secondary particles. A ligand may be imparted to the surface of the primary particle or the secondary particle. In order to improve characteristics such as light emission characteristics, dispersibility and film formability of the chalcogenide perovskite nanoparticles, other materials may be added as an additive to the powder of the chalcogenide perovskite nanoparticles.

In addition, the use of the chalcogenide perovskite nanoparticle powder is not particularly limited. For example, a solution may be prepared by being dispersed in a solvent, a composite may be prepared by being dispersed in a resin or a solid medium, a sputtering target may be used as a sintered body, or a powder may be used as a source of a vapor deposition source or the like.

(Solution)

The solution is a solution in which the chalcogenide perovskite nanoparticles are dispersed in a solvent. The term "dispersed" refers to a state in which chalcogenide perovskite nanoparticles are suspended or floating in the solvent, with some particles possibly settling. In addition, a ligand may be imparted to the surface of the primary particle or the secondary particle.

As the solvent of the solution, one or more types of solvents may be used. Examples of the type of the solvent include, but are not limited to, those described below.

Water; esters such as methyl formate, ethyl formate, propyl formate, pentyl formate, methyl acetate, ethyl acetate, and pentyl acetate; ketones such as γ-butyrolactone, acetone, dimethyl ketone, diisobutyl ketone, cyclopentanone, cyclohexanone, and methylcyclohexanone; ethers such as diethyl ether, methyl-tert-butyl ether, diisopropyl ether, dimethoxymethane, dimethoxyethane, 1,4-dioxane, 1,3-dioxolane, 4-methyldioxolane, tetrahydrofuran, methyltetrahydrofuran, anisole, and phenetole; alcohols such as methanol, ethanol, 1-propanol, 2-propanol, 1-butanol, 2-butanol, tert-butanol, 1-pentanol, 2-methyl-2-butanol, methoxypropanol, diacetone alcohol, cyclohexanol, 2-fluoroethanol, 2,2,2-trifluoroethanol, and 2,2,3,3-tetrafluoro-1-propanol; glycol ethers such as ethylene glycol monomethyl ether, ethylene glycol monoethyl ether, ethylene glycol monobutyl ether, ethylene glycol monoethyl ether acetate, and triethylene glycol dimethyl ether, organic solvents having an amide group such as N-methyl-2-pyrrolidone, N,N-dimethylformamide, acetamide, and N,N-dimethylacetamide; organic solvents having a nitrile group such as acetonitrile, isobutyronitrile, propionitrile, and methoxyacetonitrile; organic solvents having a carbonate group such as ethylene carbonate and propylene carbonate; organic solvents having a halogenated hydrocarbon group such as methylene chloride and chloroform; organic solvents having a hydrocarbon group such as n-pentane, cyclohexane, n-hexane, benzene, toluene, and xylene; and dimethyl sulfoxide.

In addition, an acid, a base, a binder material, or the like may be added to the above solution as an additive in order to improve characteristics such as light emission characteristics, dispersibility of chalcogenide perovskite nanoparticles, and film formability.

The use of the solution is not particularly limited. For example, it may be used for film formation by a coating method, a spraying method, a doctor blade method (other solution film formation methods), preparation of a composite by a composite with a solid dispersion medium, or preparation of a device using them.

(Thin Film)

The thin film is a state in which chalcogenide perovskite nanoparticles are aggregated in a planar shape. A ligand may be imparted to the surface of the primary particle or the secondary particle. In order to improve characteristics such as light emission characteristics and dispersibility of the chalcogenide perovskite nanoparticles, another material may be added to the thin film as an additive.

The method for producing the thin film is not particularly limited. Examples of the production method include a coating method, a spraying method, a doctor blade method, an inkjet method, and other methods by solution film formation; and examples thereof include a production method using a vacuum process such as a sputtering method or a vacuum vapor deposition method. In addition, chalcogenide perovskite nanoparticles may be applied, formed into a film by another method, and then subjected to firing or another processes, so that the particle shape is not maintained.

(Sheet)

The sheet is in a state in which the dispersion medium in which the chalcogenide perovskite nanoparticles are dispersed is planar. A ligand may be imparted to the surface of the primary particle or the secondary particle.

As the material used as the dispersion medium of the sheet, a polymer well known to those skilled in the art that can be used for this type of purpose can be arbitrarily applied. In a suitable embodiment, polymers of this kind are substantially translucent or substantially transparent.

For example, polymers applicable as a dispersion medium of a sheet include polyvinyl butyral: polyvinyl acetate, silicone, and derivatives of silicone, but are not limited thereto. In addition, derivatives of silicone include, but are not limited to, polyphenylmethylsiloxane, polyphenylalkylsiloxane, polydiphenylsiloxane, polydialkylsiloxane, fluorinated silicones, vinyl- and hydride-substituted silicones, ionomers, polyethylene, polyvinyl chloride, polyvinylidene chloride, polyvinyl alcohol, polypropylene, polyester, polycarbonate, polystyrene, polyacrylonitrile, ethylene-vinyl acetate copolymer, ethylene-vinyl alcohol copolymer, ethylene-methacrylic acid copolymer film, and nylon.

In order to improve characteristics such as light emission characteristics and dispersibility of the chalcogenide perovskite nanoparticles, silica fine particles or other materials such as the solvent described in the above solution may be added to the sheet as an additive.

The production method of the sheet is not particularly limited. For example, a sheet may be produced by kneading and stretching a powder and a dispersion medium, or a sheet may be produced by mixing and applying an ink containing chalcogenide perovskite nanoparticles and a dispersion medium or a precursor thereof.

(Device)

As applications of the chalcogenide perovskite nanoparticles, the powders, solutions, films, and sheets described above, down-conversion of ultraviolet light, blue light, or the like in various devices and application to light-receiving materials are assumed.

Examples of the type of device include a light emitting device such as an LED or an organic EL, a display device including the light emitting device, a lighting device including the light emitting device, an image sensor, a photoelectric conversion device, a bioluminescent label, and the like.

Examples

Synthesis Example 1

(Synthesis of Ba Diisobutyl Dithiocarbamate)

Barium hydroxide (5140 mg) was weighed in a 100 ml three-necked flask, 50 ml of pure water was added thereto, and the flask was sealed and then stirred.

Next, diisobutylamine (10.39 ml) was then added to the three-necked flask, and the mixture was further stirred for 10 minutes. Then, carbon disulfide (3.63 ml) was added thereto, and the mixture was stirred for 3 hours. It was confirmed that a white solid was gradually precipitated by stirring.

The precipitated solid was collected by filtration, washed with hexane, and then vacuum-dried to obtain a synthetic substance containing Ba diisobutyl dithiocarbamate. The yield was about 70%.

Synthesis Example 2

(Synthesis of Zr Diisobutyl Dithiocarbamate)

Zirconium chloride (2913 mg) was weighed in a 200 ml three-necked flask, and 75 ml of THF was added to hermetically seal the vessel, then the mixture was stirred for about 15 minutes.

Next, diisobutylamine (29.2 ml) was then added to a three-necked flask, and the mixture was stirred for 10 minutes. Then, carbon disulfide (10.2 ml) was further added thereto, and the mixture was further stirred for 3 hours.

After completion of stirring, the precipitated solid was filtered to recover the filtrate, and the filtrate was transferred to another container.

Subsequently, the solvent was removed from the recovered filtrate by a known method, and then the obtained residue was dispersed in hexane to obtain a white suspension.

The white suspension was filtered and the obtained solid was washed with hexane and then dried in vacuo to yield a synthetic substance containing Zr diisobutyl dithiocarbamate. The yield was about 80%.

Example 1

Ba diisobutyl dithiocarbamate (527.94 mg) obtained in Synthesis Example 1 and Zr diisobutyl dithiocarbamate (908.76 mg) obtained in Synthesis Example 2 were weighed in a 50 ml three-necked flask, and 1-octadecene (5 ml) was added and stirred.

Next, the three-necked flask was moved into the draft while the internal atmosphere was maintained at $N_2$ atmosphere, and the temperature was raised to 310°C at a heating rate of 5°C/min, and then oleylamine (3200 mg) at normal temperature was injected with a Luer-lock syringe.

Next, the solution in the three-necked flask was stirred for 18 hours while being maintained at 310°C, and then cooled to room temperature. The solution in the flask was divided into two portions. Butanol was added to one of the solutions to precipitate the mixture, and the mixture was recovered by centrifugation to obtain a powder of a synthetic substance. Toluene and ethanol were added to the other solution to precipitate, and centrifugation was performed. Then, the supernatant was discarded, and the residue was dispersed in toluene to obtain a dispersion liquid of a synthetic substance.

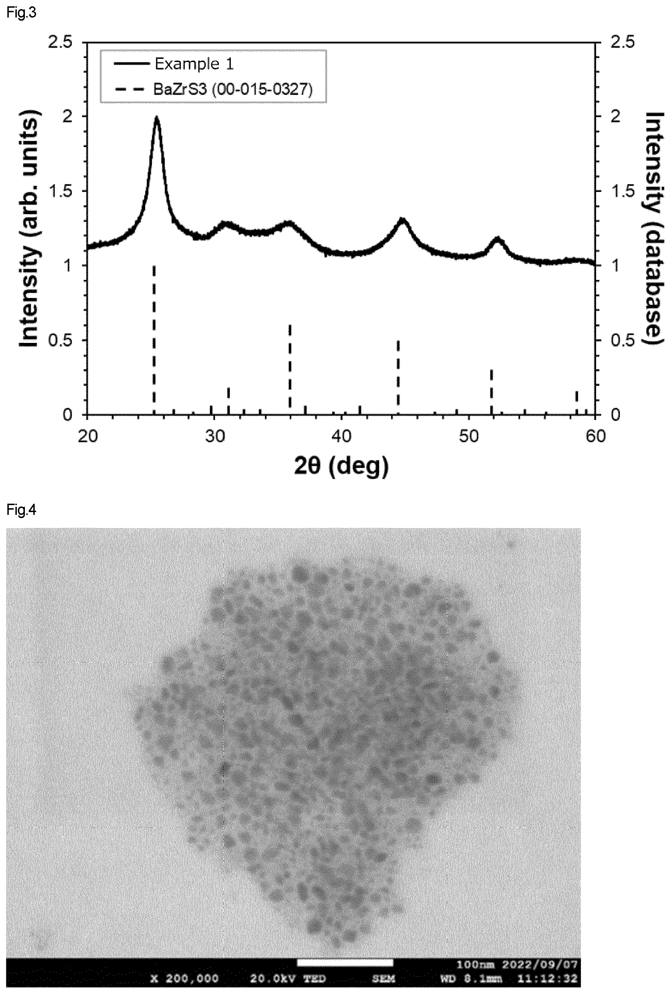

The X-ray diffraction spectrum of the synthetic substance obtained in Example 1 was measured under the following conditions. The results are illustrated in Fig. 3.

[X-ray Diffraction Analysis]

X-ray wavelength: 1.5418 Å (CuK$\alpha$-ray)
Measurement range: 5 to 60 (deg)
Measurement method: $2\theta$-$\theta$ method

Measurement sample: A sample powder of a synthetic substance was filled in a glass sample holder with a recess, and the surface was leveled using a glass plate, and the measurement was performed.

In the synthetic substance obtained in Example 1, it was confirmed that peak positions showing the top five peak intensities in the X-ray diffraction analysis spectrum were almost matched with those of $BaZrS_3$ (GdFeO$_3$ type orthorhombic perovskite) synthesized by solid phase synthesis based on JCPDS card (No. 00-015-0327). From this, it is found that the synthetic substance obtained in Example 1 is $BaZrS_3$.

The average particle diameter of the dispersion liquid of the synthetic substance obtained in Example 1 was measured by a microscope method. The microscope method refers to a method of measuring by directly measuring the size and number of particles on an image of an optical microscope or an electron microscope. Examples of the diameter used as an index in evaluating the particle diameter include a Feret's diameter, a Heywood diameter, a Martin diameter, and a kurmbein diameter. From the viewpoint of ease of calculation by image analysis, the diameter evaluated using the Heywood diameter as an index is adopted as the particle diameter.

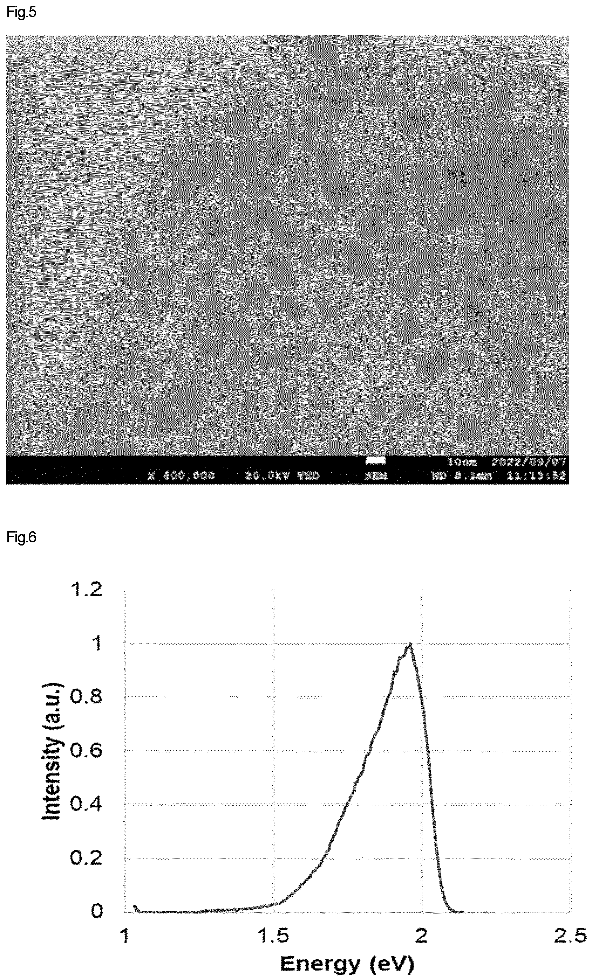

Using a scanning transmission electron microscope (STEM) ("JSM -7610 F" manufactured by JEOL Ltd.), the dispersion liquid of the synthetic substance obtained in Example 1 was observed at a magnification of 5 kV at an acceleration voltage of 200,000 times or 400,000 times. STEM images obtained by the observation are illustrated in Fig. 4 (magnification: 200,000 times) and Fig. 5 (magnification: 400,000 times).

The average of the Heywood diameters obtained by the microscope method from Figs. 4 and 5 was 13.5 nm.

The fluorescence spectrum of the dispersion liquid of the synthetic substance obtained in Example 1 was measured under the following conditions. The results are illustrated in Fig. 6. The actual measured value of the light emission peak energy, that is, the band gap was 1.96 eV. The actual measured value of the band gap coincides with the band gap unique to the semiconductor of $BaZrS_3$ obtained by Formula (A). The band gap ($E_{g,bulk}$) unique to the semiconductor calculated by Formula (A) is $1.9485 \pm 0.0974$ eV(($E_{g,calc(HSE06)}$)=1.77 eV).

[Fluorescence Spectrum Measurement]

Measuring device: PL lifetime measuring device (Hamamatsu Photonics K.K., C12132)
Excitation light wavelength: 532 nm
Measurement range: 580 to 1200 nm
Measurement sample: A quartz cell was filled with a dispersion liquid, and the dispersion liquid was placed in a sample holder in the device to perform measurement.

Industrial Applicability

The chalcogenide perovskite of the present invention, as well as powders, solutions, films, and sheets containing the chalcogenide perovskite, can be applied to light emitting materials and wavelength conversion elements in light emitting devices such as displays, as well as photoelectric conversion elements such as solar cells and sensors.

Although several embodiments and/or examples of the present invention have been described in detail above, those skilled in the art will readily understand that numerous modifications can be made to these exemplary embodiments and/or examples without substantially departing from the novel teachings and effects of the present invention. Therefore, many of these modifications fall within the scope of the present invention.

All literature cited in this specification, as well as the contents of the applications on which the priority claim under the Paris Convention is based, are incorporated herein by reference in their entirety.

**Claims**

1. A chalcogenide perovskite having an average particle size of less than 40 nm and an actual measured value of a band gap that matches a range of a band gap ($E_{g,bulk}$) unique to the semiconductor calculated by Formula (A) below based on a band gap calculated value ($E_{g,calc(HSE06)}$) obtained by a density functional theory using a hybrid functional (HSE06).

[Math. 3]

$$E_{g,bulk} = \left(0.9286 \times E_{g,calc(HSE06)} + 0.3049\right) \times \left(1 \pm 0.05\right) \quad \cdots \text{(A)}$$

2. The chalcogenide perovskite according to claim 1, wherein the actual measured value of the band gap is more than 0 eV and less than 2.09 eV.

3. The chalcogenide perovskite according to claim 1 or 2, wherein the actual measured value of the band gap is 1.0 eV or more and 2.0 eV or less.

4. The chalcogenide perovskite according to any one of claims 1 to 3, wherein the actual measured value of the band gap is 1.8 eV or more and 2.0 eV or less.

5. The chalcogenide perovskite according to any one of claims 1 to 4, wherein the average particle size is 4 nm or more and less than 40 nm.

6. The chalcogenide perovskite according to claim 5, wherein the average particle size is 5 nm or more.

7. The chalcogenide perovskite according to claim 6, wherein the average particle size is 6 nm or more.

8. The chalcogenide perovskite according to any one of claims 5 to 7, wherein the average particle size is less than 35 nm.

9. The chalcogenide perovskite according to claim 8, wherein the average particle size is less than 32 nm.

10. The chalcogenide perovskite according to claim 9, wherein the average particle size is less than 30 nm.

11. The chalcogenide perovskite according to any one of claims 1 to 10, wherein the chalcogenide perovskite has a composition represented by Formula (101) or (102) below:

$ABCh_3$ ...           (101)

$A'_2A_{n-1}B_nCh_{3n+1}$ ...         (102)

(In Formulae (101) and (102), A and A each represent Ca, Mg, Sr, Ba, or a combination thereof; B represents Ti, Zr, Hf, or a combination thereof; and Ch represents S, Se, Te, or a combination thereof. In Formula (102), n represents an integer of 1 or more and 10 or less. In Formula (102), A and A may be the same as or different from each other. The chalcogenide perovskite may be a solid solution in which a part or all of A, A, B, and Ch are substituted with another element in the composition represented by Formula (101) or (102).)

12. The chalcogenide perovskite according to claim 11, wherein n is 1 or more and 3 or less.

13. The chalcogenide perovskite according to claim 11 or 12, wherein a composition of the chalcogenide perovskite is $BaZrS_3$.

14. A powder comprising:
the chalcogenide perovskite according to any one of claims 1 to 13.

15. A solution comprising:
the chalcogenide perovskite according to any one of claims 1 to 13.

16. A thin film comprising:
the chalcogenide perovskite according to any one of claims 1 to 13.

**17.** A sheet comprising:
the chalcogenide perovskite according to any one of claims 1 to 13.

**18.** A device comprising:
the chalcogenide perovskite according to any one of claims 1 to 13.

**19.** The device according to claim 18, wherein the device is a photoelectric conversion element.

Fig.1

| Formula | Exp | Calc(HSE06) |
|---|---|---|
| $Ba(Zr_{0.95}Ti_{0.05})S_3$ | 1.63[1] | 1.43[2] |
| $BaZrS_3$ | 1.94[1] | 1.77[1] |
| $BaHfS_3$ | 2.17[1], 2.06[3] | 2.01[1], 1.90[3] |
| $SrZrS_3$ | 2.14[1] | 1.94[1] |
| $SrHfS_3$ | 2.41[1], 2.32[3] | 2.27[1], 2.18[3] |

Fig.2

$y = 0.9286x + 0.3049$

Fig.3

Fig.4

Fig.5

Fig.6

## INTERNATIONAL SEARCH REPORT

| International application No. |
| --- |
| **PCT/JP2023/035933** |

| A. | CLASSIFICATION OF SUBJECT MATTER |
| --- | --- |

*C01G 25/00*(2006.01)i; *C09K 11/67*(2006.01)i; *C09K 11/88*(2006.01)i
FI: C01G25/00; C09K11/67; C09K11/88

According to International Patent Classification (IPC) or to both national classification and IPC

| B. | FIELDS SEARCHED |
| --- | --- |

Minimum documentation searched (classification system followed by classification symbols)

C01G25/00; C09K11/67; C09K11/88

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JSTChina/JST7580 (JDreamIII); CAplus/REGISTRY (STN)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | TURNLEY, Jonathan W. et al. Solution Deposition for Chalcogenide Perovskites: A Low-Temperature Route to BaMS3 Materials (M = Ti, Zr, Hf). Journal of the American Chemical Society. 29 September 2022, 144, pp. 18234-18239<br>p. 18234, left column, lines 23-26, p. 18236, left column, lines 14-18, p. 18236, right column, lines 35-38, p. 18237, left column, lines 3-10, fig. 5(c) | 1-11, 16-19 |
| A | entire text, all drawings | 12-15 |
| A | YANG, Ruiquan et al. Low-temperature, Solution-Based Synthesis of Luminescent Chalcogenide Perovskite BaZrS3 Nanoparticles. Journal of the American Chemical Society. 24 August 2022, 144, pp. 15928-15931<br>entire text, all drawings | 1-19 |
| A | US 2019/0225883 A1 (SAMSUNG ELECTRONICS CO., LTD.) 25 July 2019 (2019-07-25)<br>entire text, all drawings | 1-19 |
| E, A | US 2023/0322576 A1 (PURDUE RESEARCH FOUNDATION) 12 October 2023 (2023-10-12)<br>entire text, all drawings | 1-19 |

☑ Further documents are listed in the continuation of Box C.    ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **05 December 2023** | **19 December 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2023/035933** |

**C.  DOCUMENTS CONSIDERED TO BE RELEVANT**

| Category* | Citation of document, with indication, where appropriate, of the relevant passages | Relevant to claim No. |
| --- | --- | --- |
| P, X | WO 2023/100919 A1 (IDEMITSU KOSAN CO) 08 June 2023 (2023-06-08) paragraphs [0092]-[0095] | 1-19 |

Form PCT/ISA/210 (second sheet) (January 2015)

**INTERNATIONAL SEARCH REPORT**
**Information on patent family members**

International application No.

**PCT/JP2023/035933**

| Patent document cited in search report | | | Publication date (day/month/year) | Patent family member(s) | Publication date (day/month/year) |
|---|---|---|---|---|---|
| US | 2019/0225883 | A1 | 25 July 2019 | CN 110065931 A<br>entire text, all drawings<br>KR 10-2019-0089779 A | |
| US | 2023/0322576 | A1 | 12 October 2023 | (Family: none) | |
| WO | 2023/100919 | A1 | 08 June 2023 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Colloidal BaZrS3 chalcogenide perovskite nanocrystals for thin film device fabrication. **V. K. RAVI et al.** Nanoscale. The Royal Society of Chemistry, 2021, vol. 13, 1616-1623
- **R. YANG et al.** Low-Temperature, Solution-Based Synthesis of Luminescent Chalcogenide Perovskite BaZrS3 Nanoparticles. *J.Am. Chem. Soc.*, 2022, vol. 144 (35), 15928-15931
- **Y NISHIGAKI et al.** Extraordinary Strong Band-Edge Absorption in Distorted Chalcogenide Perovskites. *Sol. RPL*, 2020, 1900555

- **S. SHARMA et al.** Bandgap Tuning in BaZrS3 Perovskite Thin Films. *ACS Appl. Electron. Mater.*, 2021, vol. 3 (8), 3306-3312
- **K HANAZAWA et al.** Material Design of Green-Light-Emitting Semiconductors: Perovskite-Type Sulfide SrHfS3. *J. Am. Chem. Soc.*, 2022, vol. 141 (13), 5343-5349
- **KIYOSHI KANIE et al.** Liquid Phase Synthesis of Functional Inorganic Nanoparticles Controlled in Size and Shape and Application to Hybrid Materials by the Surface Modification. *Journal of the Japanese Association for Crystal Growth*, 2017, vol. 44 (2), 66-73